# EUROPÄISCHE PATENTSCHRIFT

(11) **EP 4 165 457 B1**
(45) Veröffentlichungstag und Bekanntmachung des Hinweises auf die Patenterteilung: **29.07.2026**
(21) Anmeldenummer: 21762121.8
(22) Anmeldetag: 19.08.2021
(51) Int. Cl.: G02B 23/24, A61B 1/00, A61B 1/06

(54) **BELEUCHTUNGSVORRICHTUNG FÜR EIN ENDOSKOP**
ILLUMINATION DEVICE FOR AN ENDOSCOPE
DISPOSITIF D'ÉCLAIRAGE POUR ENDOSCOPE

(30) Priorität: 31.08.2020 DE 102020122636
(43) Veröffentlichungstag der Anmeldung: 19.04.2023
(73) Patentinhaber: HOYA CORPORATION, Shinjuku-ku Tokyo 160-8347 (JP)
(72) Erfinder: LANDES, Hermann, 86316 Friedberg (DE)
(74) Vertreter: Lucke, Andreas
(86) Internationale Anmeldenummer: PCT/IB2021/057629
(87) Internationale Veröffentlichungsnummer: WO 2022/043835

(56) Entgegenhaltungen:
- EP-A1- 1 769 718
- DE-A1- 102018 202 243
- US-A1- 2001 003 142
- US-A1- 2009 069 633
- US-A1- 2011 157 574
- US-A1- 2014 357 948

## Beschreibung

Die vorliegende Erfindung bezieht sich auf eine Beleuchtungsvorrichtung für ein Endoskop. Insbesondere bezieht sie sich auf eine Beleuchtungsvorrichtung für ein Endoskop mit einem Objektiv, das einen großen Blickwinkel, insbesondere einen Blickwinkel, der größer als 180° ist, hat.

### Stand der Technik

Das Beleuchtungssystem eines Endoskops kann mehrere LEDs aufweisen, die um ein Objektiv angeordnet sind. Die Lichtemissionseinrichtungen sind dabei typischerweise in einer Ebene angeordnet, die senkrecht zur optischen Achse des Objektivs ist, und die Lichtemissionseinrichtungen sind so eingerichtet, dass sie parallel zur optischen Achse Licht abstrahlen. Genauer ausgedrückt liegt der Schwerpunkt der Winkelverteilung der Abstrahlung in Richtung der optischen Achse. Das Licht der LEDs tritt durch eine durchsichtige Kappe und beleuchtet dann das Blickfeld. Die durchsichtige Kappe begrenzt die Beleuchtungsvorrichtung zum Objektraum, der das Blickfeld des Objektivs enthält, hin.

Aus der US 2011/0157574 A1 ist ein Endoskop mit einem optischen Beleuchtungssystem bekannt, wobei an dem distalen Ende des Endoskops ein Schulterabschnitt sowie eine plano-konkave Linse angeordnet sind, deren Oberflächen derart geneigt sind, dass der Schulterabschnitt und die Linse in Richtung der Spitze des Endoskops schmaler werden.

Die US 2014/0357948 A1 beschreibt ein optisches Beleuchtungssystem für ein Endoskop, welches eine optische Faser sowie einen ersten Spiegel und einen zweiten Spiegel aufweist, wobei letztere gegenüber einem Emissionsende der optischen Faser angeordnet sind und dazu eingerichtet sind, das aus dem Emissionsende emittierte Licht aufzuweiten.

Die DE 10 2018 202 243 A1 offenbart ein medizinisch-endoskopisches Instrument mit einem distalen länglichen Einführabschnitt zum minimalinvasiven Einführen in einen menschlichen oder tierischen Körper, wobei der Einführabschnitt mindestens eine erste LED, eine zweite LED und einen Bildsensor aufweist, wobei die erste LED, die zweite LED und der Bildsensor in eine gemeinsame Blickrichtung ausgerichtet sind. Die erste LED weist dabei ein für eine Fluoreszenz-Endoskopie geeignetes erstes Leuchtspektrum und die zweite LED ein für eine Weißlicht-Endoskopie geeignetes zweites Leuchtspektrum auf, wobei ein Lichtfilter in Blickrichtung vor der zweiten LED angeordnet ist. Das Lichtfilter weist dabei ein Transmissionsspektrum auf und die zweite LED ist dazu ausgestaltet, gemäß dem zweiten Leuchtspektrum in einem ersten Wellenlängenbereich im Mittel intensiver zu strahlen als in einem zweiten Wellenlängenbereich, und wobei das Lichtfilter dazu ausgestaltet ist, von der zweiten LED ausgestrahltes Licht gemäß dem Transmissionsspektrum im zweiten Wellenlängenbereich im Mittel weniger durchzulassen als im ersten Wellenlängenbereich.

Aus der EP 1 769 718 A1 ist ein Aufsatz für ein Endoskop bekannt, der es dem Endoskop ermöglicht, Bilder von Bereichen vor dem Endoskop und an den Seiten des Endoskops aufzunehmen. Der Aufsatz weist ein transparentes Befestigungsteil, ein transparentes zylindrisches Bildaufnahmeteil, einen trompeten-förmigen ersten Spiegel an der äußeren Wand des Bildaufnahmeteils sowie eine Weitwinkellinse mit einem Hyperboloid auf, wobei an einem Teil des Hyperboloids der Weitwinkellinse ein zweiter Spiegel ausgebildet ist.

Die US 2001/0003142 A1 beschreibt eine Endoskopvorrichtung mit einem optischen Belichtungssystem mit einer größeren Beleuchtungsstärke oder breiteren Lichtverteilung auf beiden Seiten eines Endes eines Einführungsteils in Richtung einer langen Seite eines Bildschirms und einem optischen Belichtungssystem mit einer kleineren Beleuchtungsstärke oder schmaleren Lichtverteilung auf beiden Seiten des Endes in Richtung einer kurzen Seite des Bildschirms.

Aus der US 2009/0069633 A1 ist ein Kapselendoskop bekannt, welches Lichtemittervorrichtungen aufweist, die um einen Außenumfang einer Objektivlinse und gegenüber einem Ende der Objektivlinse zurückversetzt angeordnet sind, wobei die Lichtemittervorrichtungen unter einem Winkel zu einer Mittenachse der Objektivlinse nach außen geneigt sind.

### Technisches Problem

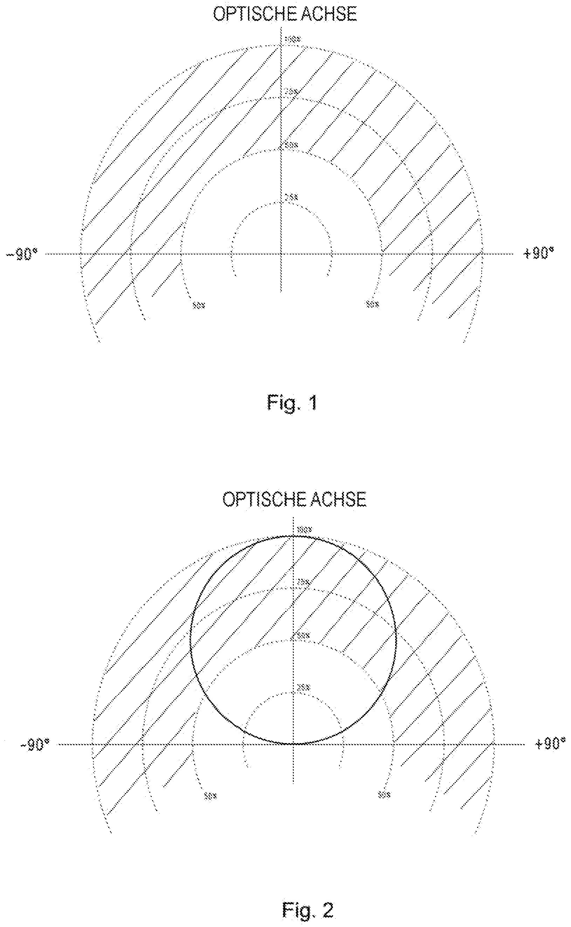

Es ist wünschenswert, dass das Blickfeld eines Objektivs eines Endoskops mit einem bildgebenden System möglichst homogen ausgeleuchtet wird, wie in Fig. 1 durch den schraffierten Bereich für einen Blickwinkel von 180° gezeigt. Wenn das Objektiv einen Blickwinkel hat, der größer als 180° ist, und die LEDs Lambertsche Strahler sind, die in Richtung der optischen Achse ihr Licht direkt auf die durchsichtige Kappe ausstrahlen, kann eine einigermaßen homogene Beleuchtung des Blickfelds nicht erreicht werden, wie in Fig. 2 gezeigt. In Fig. 2 gibt die durchgezogene Linie das Strahlungsfeld des Lambertschen Strahlers an.

### Lösung des Problems

Die vorliegende Erfindung stellt eine Endoskopspitze oder ein Kapselendoskop mit den Merkmalen des Anspruchs 1 bereit. Beispielhafte Ausgestaltungen sind in den abhängigen Ansprüchen angegeben.

Es wird bereitgestellt: Eine Endoskopspitze oder ein Kapselendoskop mit
einem Objektiv zum Abbilden eines Blickfelds; und
einer Beleuchtungsvorrichtung zum Beleuchten des Blickfelds mit Beleuchtungslicht, wobei
das Objektiv eine optische Achse hat;
das Objektiv einen Blickwinkel, der größer als 180° ist, hat;
die Beleuchtungsvorrichtung in einer Draufsicht entlang der optischen Achse um das Objektiv herum angeordnet ist;
die Beleuchtungsvorrichtung eine durchsichtige Kappe aufweist, aus der das Beleuchtungslicht in das Blickfeld emittiert wird;
die Beleuchtungsvorrichtung eine oder mehrere Lichtemissionseinrichtungen aufweist, die konfiguriert sind, jeweiliges Emissionslicht aus einer jeweiligen Lichtemissionsfläche zu emittieren; und
zumindest eine der Lichtemissionseinrichtungen so konfiguriert ist, dass ein Schwerpunkt einer Winkelverteilung des jeweiligen Emissionslichts in einer Richtung, die um einen Winkel, der größer oder gleich 5° und kleiner oder gleich 85° ist, weg von der optischen Achse gekippt ist, liegt, wobei die Beleuchtungsvorrichtung ferner für jede der Lichtemissionseinrichtungen eine jeweilige reflektierende Fläche aufweist, die zumindest einen Teil des jeweiligen Emissionslichts in eine jeweilige Richtung reflektiert, die weiter weg von der Richtung der optischen Achse ist als eine jeweilige Richtung, in der der Teil des jeweiligen Emissionslichts auf die jeweilige reflektierende Fläche trifft,
wobei die Beleuchtungsvorrichtung rotationssymmetrisch um eine Rotationsachse ist, die jeweiligen reflektierenden Flächen Teil eines Ringspiegels sind und der Ringspiegel einen Teil einer ersten Kegelstumpffläche bildet, wobei die Achse der ersten Kegelstumpffläche vorzugsweise identisch mit der optischen Achse ist.

### Vorteile der Lösung

Durch die Beleuchtungseinrichtung wird eine einigermaßen homogene Beleuchtung des Blickfelds des Objektivs erreicht, selbst wenn es einen Blickwinkel von mehr als 180° hat. "Einigermaßen homogen" bedeutet, dass die Lichtstärke über den gesamten Blickwinkel zwischen 50% und 100% der maximalen Lichtstärke ist (bevorzugt zwischen 75% und 100% der maximalen Lichtstärke). Einige Ausführungsbeispiele sind zusätzlich relativ einfach und platzsparend. Auf optisch brechende Komponenten kann in einigen Fällen verzichtet werden (wenn man eine evtl. minimale Brechung an der durchsichtigen Kappe vernachlässigt), was die Herstellung einfach und damit kostengünstig macht.

### Kurze Beschreibung der Zeichnungen

Fig. 1 zeigt ein Anforderungsprofil an die Homogenität der Beleuchtung des Blickfelds des Objektivs;
Fig. 2 zeigt, wie ein Lambertscher Strahler das Blickfeld gemäß dem Stand der Technik ausleuchtet;
Fig. 3 zeigt eine Endoskopspitze mit einem Objektiv, in der die Beleuchtungsvorrichtung nach einem Ausführungsbeispiel der Erfindung angebracht werden kann;
Fig. 4 zeigt eine Endoskopspitze gemäß dem Stand der Technik;
Fig. 5 illustriert den Blickwinkel des Objektivs, das sich in der Endoskopspitze der Fig. 4 befindet;
Fig. 6 vergleicht den Blickwinkel des Objektivs mit dem Abstrahlwinkel der Beleuchtung in der Endoskopspitze der Fig. 4;
Fig. 7 zeigt eine Draufsicht auf die LEDs der Endoskopspitze der Fig. 4;
Fig. 8 zeigt ein Strahlungsdiagramm für die Endoskopspitze der Fig. 4;
Fig. 9 zeigt eine mögliche Anordnung von LEDs in einer Endoskopspitze;
Fig. 10 zeigt ein Strahlungsdiagramm für die Endoskopspitze der Fig. 9;
Fig. 11 zeigt eine Endoskopspitze gemäß einer für das Verständnis der Erfindung nützlichen Ausführungsform;
Fig. 12 zeigt Querschnitte und Draufsichten von Komponenten der Endoskopspitze gemäß Fig. 11;
Fig. 13 vergleicht den Abstrahlwinkel der Beleuchtung und den Öffnungswinkel des Objektivs der Endoskopspitze der Fig. 11;
Fig. 14 zeigt ein Abstrahlungsdiagramm für die Endoskopspitze der Fig. 11;
Fig. 15 zeigt eine Endoskopspitze gemäß einer für das Verständnis der Erfindung nützlichen Ausführungsform;
Fig. 16 zeigt Querschnitte und Draufsichten von Komponenten der Endoskopspitze gemäß Fig. 15;
Fig. 17 vergleicht den Abstrahlwinkel der Beleuchtung und den Öffnungswinkel des Objektivs der Endoskopspitze der Fig. 15;
Fig. 18 zeigt ein Abstrahlungsdiagramm für die Endoskopspitze der Fig. 15;
Fig. 19 zeigt das Prinzip der Abschattung in einer Endoskopspitze gemäß dem Stand der Technik;
Fig. 20 zeigt, wie das abgeschattete Licht gemäß dem Ausführungsbeispiel zur Beleuchtung des äußeren Blickfelds ausgenutzt wird;
Fig. 21 zeigt eine Referenzkonfiguration, um ein Prinzip der Ausführungsbeispiele der Erfindung zu erläutern; und
Fig. 22 zeigt ein Ausführungsbeispiel der Erfindung, um das Prinzip der Ausführungsbeispiele der Erfindung zu erläutern.

### Detaillierte Beschreibung von Ausführungsbeispielen

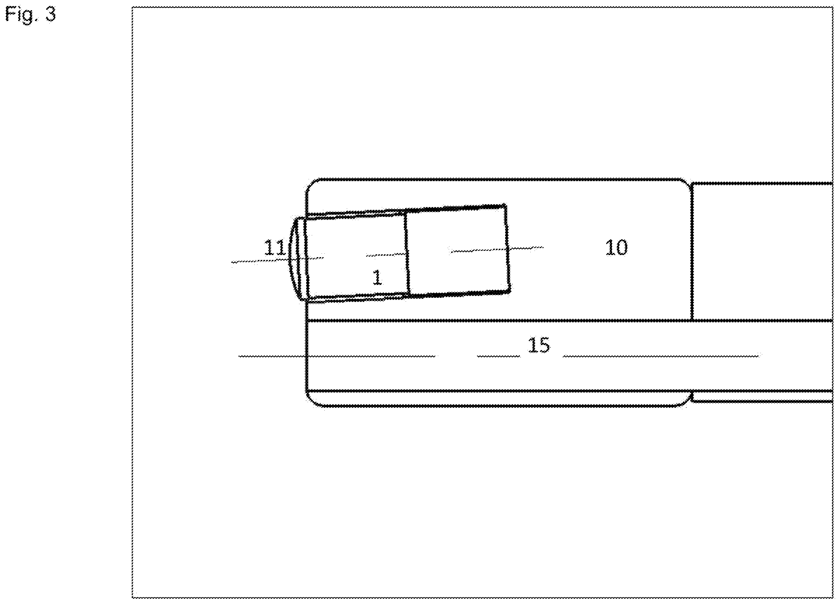

Fig. 3 zeigt ein Beispiel einer Endoskopspitze 10, in der ein Objektiv 1 (bevorzugt ein Weitwinkelobjektiv mit einem Blickwinkel von mehr als 180°) angeordnet ist. Die optische Achse 11 des Objektivs 1 kann parallel zur Symmetrieachse der Endoskopspitze 10 verlaufen. Insbesondere kann die optische Achse 11 des Objektivs 1 identisch mit der Symmetrieachse der Endoskopspitze 10 sein oder aber versetzt zu ihr sein. Die optische Achse 11 kann aber auch gegenüber der Symmetrieachse der Endoskopspitze 10 geneigt sein, wie in dem Beispiel von Fig. 3 gezeigt.

Die Beleuchtungsvorrichtung ist in einer Draufsicht auf das distale Ende der Endoskopspitze um das Objektiv 1 herum angebracht. Sie kann z.B. direkt um das Objektiv 1 herum angebracht sein oder von dem Objektiv 1 beabstandet sein. Die Beleuchtungsvorrichtung kann rotationssysmmetrisch sein. Wenn die Beleuchtungsvorrichtung rotationssymmetrisch ist, ist ihre Rotationsachse bevorzugt (aber nicht notwendigerweise) identisch mit der optischen Achse 11 des Objektivs 1.

Im weiteren wird die Erfindung anhand einer Endskopspitze 10 erklärt, bei der das Objektiv 1 symmetrisch zur Achse der Endoskopspitze 10 angebracht ist und die Beleuchtung rotationssymmetrisch um die optische Achse des Objektivs 1 angebracht ist. Jedoch ist die Erfindung nicht auf diese spezielle Konfiguration beschränkt, wie oben erläutert.

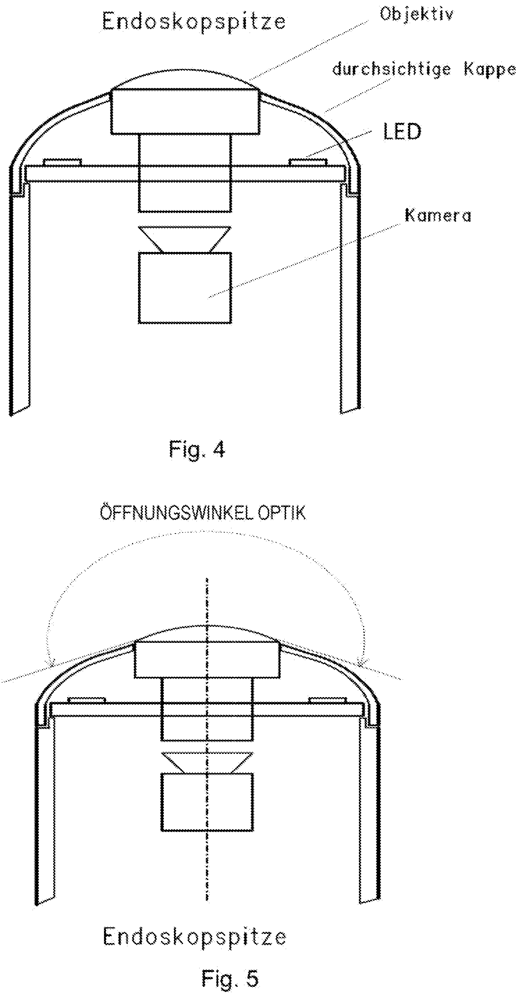

Fig. 4 zeigt eine Endoskopspitze nach dem Stand der Technik. In der Endoskopspitze befindet sich ein Objektiv mit einem Blickwinkel von mehr als 180°. Der Blickwinkel ("Öffnungswinkel Optik") ist in Fig. 5 illustriert. Wegen des großen Öffnungswinkels der Optik sollte sich das Objektiv an der Spitze des Endoskops befinden und alle anderen Teile (Endoskopkappe, Beleuchtung) sind hinter diesem zurückgesetzt, um nicht im Blickfeld zu erscheinen.

Die Endoskopspitze kann ferner eine Kamera enthalten, um das von dem Objektiv aufgenommene Bild des Blickfelds aufzunehmen. Die Kamera kann z.B. einen CCD Chip oder CMOS Chip als Sensor aufweisen. Anstelle der Kamera kann die Endoskopspitze auch einen Teil einer Relay-Optik aufweisen, die das von dem Objektiv aufgenommene Bild an das proximale Ende des Endoskops leitet.

Die Endoskopspitze enthält ferner eine Beleuchtungsvorrichtung, um das Blickfeld des Objektivs (bzw. den Objektraum, der das Blickfeld enthält) zu beleuchten. In der Beleuchtungsvorrichtung sind LEDs angebracht, die den Objektraum durch eine durchsichtige Kappe (Endoskopkappe) hindurch beleuchten. Die Kappe ist durchsichtig, wenn ihre Transparenz mindestens 75%, bevorzugt mindestens 90%, und noch bevorzugter mindestens 95% für alle Wellenlängen, die mit einer Intensität von mindestens 50% der Maximalintensität der LEDs in Abhängigkeit von der Wellenlänge abgestrahlt werden. Die Kappe sollte nicht tönend sein. Das heißt, dass eine Transparenz für verschiedene Wellenlängen sich um nicht mehr als 20%, bevorzugt um nicht mehr als 10% unterscheidet. Wenn die LEDs auch Licht mit einer Wellenlänge außerhalb des sichtbaren Bereichs (400 nm bis 800 nm) ausstrahlen, können die obigen Bedingungen auf die Wellenlängen im sichtbaren Bereich und nicht auf die Wellenlängen außerhalb des sichtbaren Bereichs angewendet werden.

Die Brechkraft der Kappe ist typischerweise klein, da sie im Wesentlichen wie eine planparallele Platte wirkt. Die Brechkraft kann lokal verschieden sein. Z.B. kann die maximale lokale Brechkraft 2 dpt (2 m⁻¹), bevorzugt 1 dpt (1 m⁻¹), und noch bevorzugter 0.5 dpt (0.5 m⁻¹) sein. Jedoch kann in einigen Ausführungsbeispielen der Erfindung die Kappe als Linse mit einer Brechkraft ausgestaltet sein, um das Beleuchtungslicht in vorbestimmte Bereiche des Blickfelds zu richten.

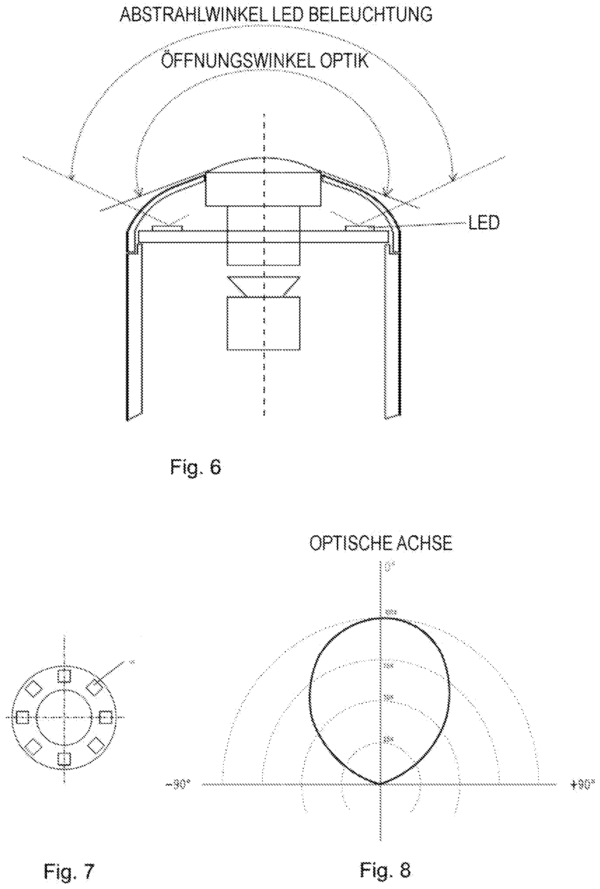

Die LEDs können alle vom gleichen Typ sein, oder von verschiedenen Typen sein. Z.B. können zwei verschiedene Typen verwendet werden, um den Objektraum mit zwei verschiedenen Farben zu beleuchten. In diesem Fall sollten die LEDs jedes einzelnen Typs bevorzugt rotationssymmetrisch um die optische Achse des Objektivs angebracht sein. Ein Beispiel dazu ist in Fig. 7 in der Draufsicht gezeigt.

Die LEDs können alle einzeln oder in Gruppen steuerbar sein. "Steuerbar" bedeutet zumindest, dass die LEDs ein- und ausschaltbar sind. In einigen Ausführungsbeispielen kann auch die Intensität und/oder die Farbe des Emissionslichts gesteuert werden.

Fig. 6 zeigt den Abstrahlwinkel der LED Beleuchtung der Endoskopspitze der Fig. 4 gemäß dem Stand der Technik. In diesem Fall ist der Abstrahlwinkel der LED Beleuchtung so, dass das Blickfeld des Objektivs an den Rändern nicht oder nicht ausreichend ausgeleuchtet wird. Das heißt, der große Blickwinkel des Objektivs ist (ziemlich) nutzlos. Der Abstrahlwinkel gibt den Bereich an, innerhalb dessen die Intensität des Lichts mindestens 50% der Maximalintensität (bei gleichem Abstand von der LED) ist. Fig. 8 zeigt ein Strahlungsdiagramm entsprechend Fig. 6. Alle gezeigten Strahlungsdiagramme sind auf die optische Achse des Objektivs bezogen. In allen gezeigten Strahlungsdiagrammen ist die Gesamtbeleuchtung auf 100% normiert.

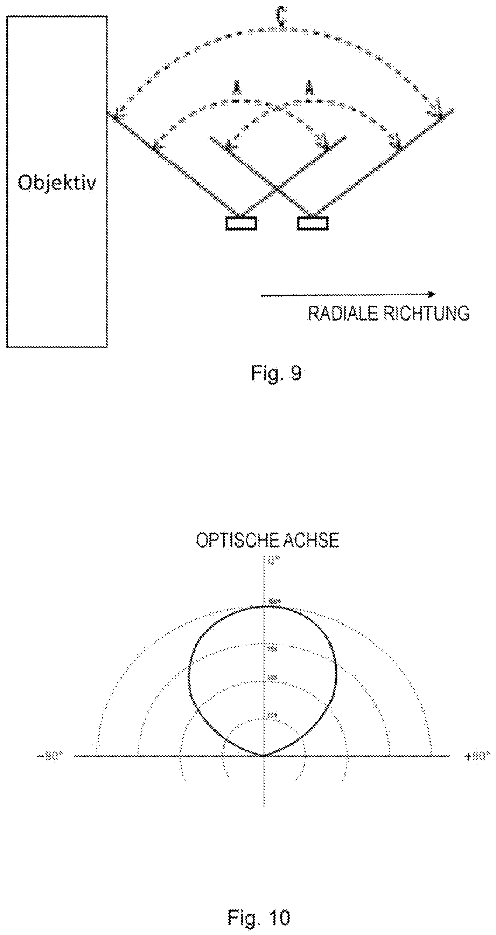

Man könnte versuchen, mehrere LEDs des gleichen Typs nebeneinander in radialer Richtung anzubringen, wie in Fig. 9 gezeigt. Jedoch ändert sich dadurch nicht der Abstrahlwinkel (C = A), sodass immer noch ein Teil des äußeren Blickfelds unbeleuchtet bleibt. Fig. 10 zeigt das entsprechende Strahlungsdiagramm, das sich qualitativ nicht von dem in Fig. 8 gezeigten Strahlungsdiagramm unterscheidet. Im Prinzip entspricht das Anbringen mehrerer LEDs in radialer Richtung, die jeweils parallel zur optischen Achse des Objektivs emittieren, nur einer Vergrößerung der Emissionsfläche einer einzelnen LED.

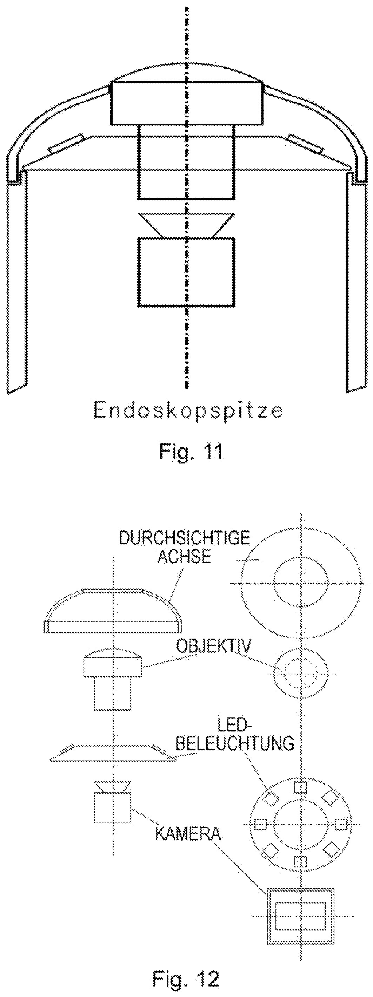

Fig. 11 zeigt eine Endoskopspitze gemäß einer für das Verständnis der Erfindung nützlichen Ausführungsform. Die Endoskopspitze entspricht genau der Endoskopspitze der Fig. 4, außer dass die LEDs nicht parallel zur optischen Achse des Objektivs abstrahlen, sondern mit einem endlichen Winkel davon weg abstrahlen. Der endliche Winkel kann zum Beispiel in einem Bereich von 5° bis 80°, 10° bis 85°, oder 10° bis 80°, bevorzugt in einem Bereich von 5° bis 50°, und noch bevorzugter in einem Bereich von 10° bis 45° liegen. Der endliche Winkel ist verschieden von 0° und von 90°.

Dazu sind die LEDs auf einer Fläche angebracht, die gegenüber einer Fläche, die senkrecht zur optischen Achse ist, geneigt ist. Der Neigungswinkel der Fläche entspricht der Änderung des Abstrahlwinkels der Beleuchtungsvorrichtung und kann gemäß der gewünschten Abstrahlcharakteristik gewählt werden. Die Fläche ist durch einen Kegelstumpf gebildet, der anstelle der ebenen Montagefläche für die LEDs gemäß Fig. 4 eingesetzt sein kann. Bevorzugt (aber nicht notwendigerweise) fällt die Achse des Kegelstumpfs mit der optischen Achse des Objektivs zusammen.

Gemäß der Fig. 11 sind die LEDs durch die Anbringung auf der Kegelstumpffläche auch näher am distalen Ende. Dies ist jedoch nicht notwendig. Zum Beispiel können anstelle der Kegelstumpffläche auch Mulden in die LED-Montagefläche der Fig. 4 angebracht werden, so dass sich der Mittelpunkt der LED Emissionsfläche auf der gleichen Höhe in Richtung der optischen Achse befindet wie in Fig. 4.

Fig. 12 zeigt die Komponenten der Endoskopspitze der Fig. 11 im Querschnitt (links) und in der Draufsicht (rechts). Die Draufsicht entspricht genau der Draufsicht auf eine Endoskopspitze der Fig. 4, im Querschnitt ist lediglich der Kegelstumpf für die Montage der LEDs verschieden von dem Querschnitt der Endoskopspitze der Fig. 4.

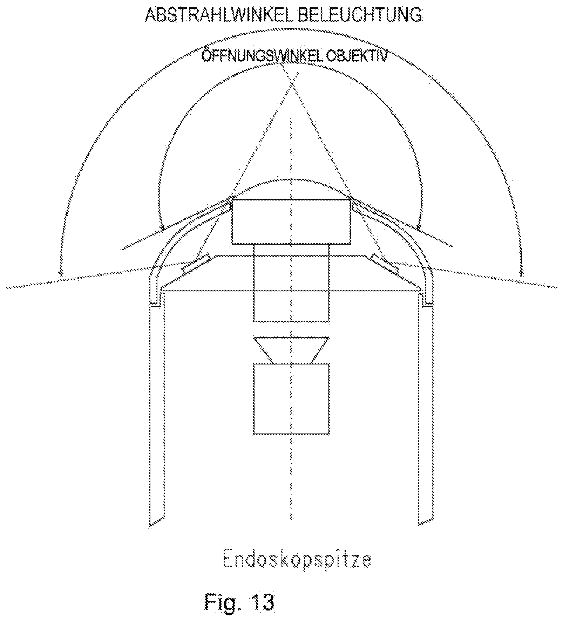

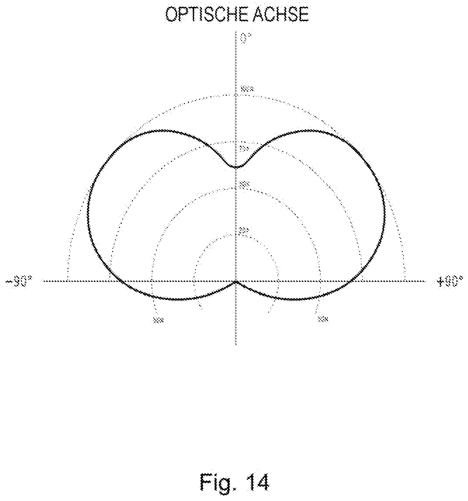

Fig. 13 zeigt den Abstrahlwinkel der Beleuchtung und den Öffnungswinkel des Objektivs der Endoskopspitze der Fig. 11, entsprechend der Darstellung in Fig. 6. Wie man sieht, ist der äußere Bereich des Blickfelds des Endoskops nun wesentlich besser beleuchtet. Fig. 14 zeigt das entsprechende Abstrahlungsdiagramm. Auch bei Blickwinkeln von mehr als 180° ist die Beleuchtung noch ausreichend.

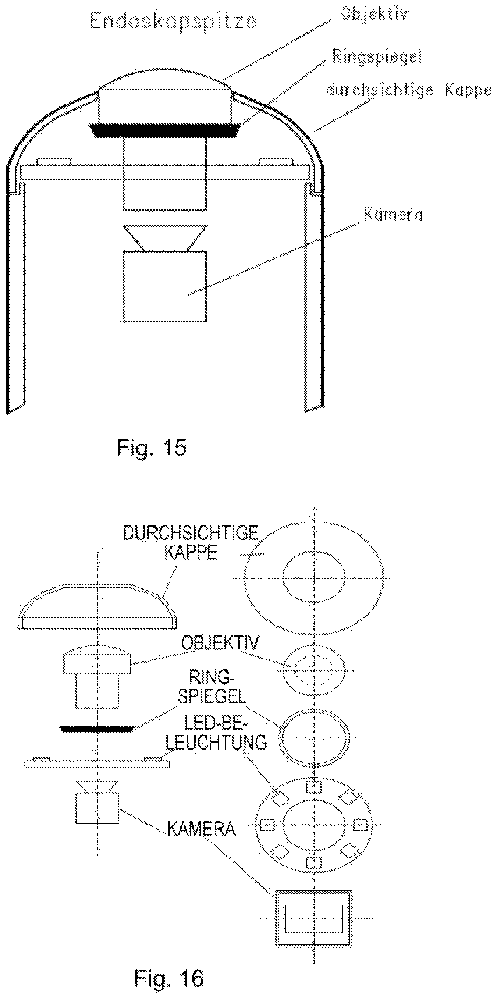

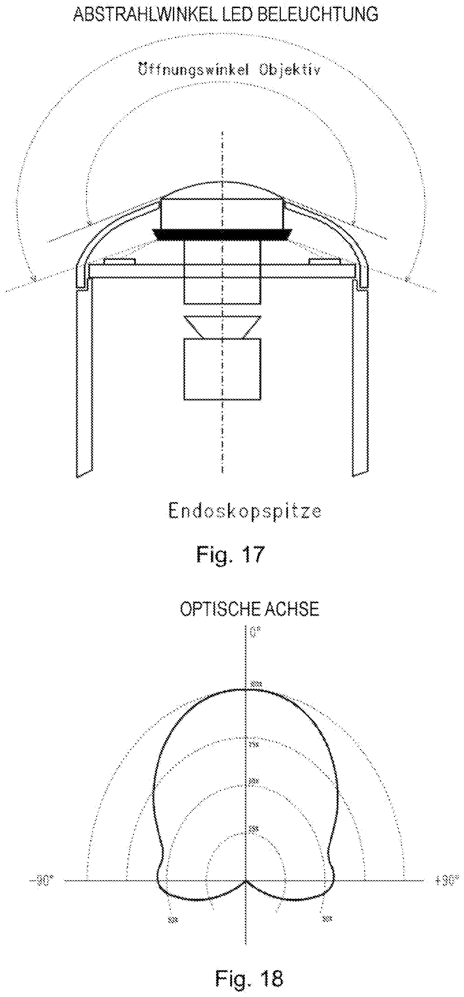

Die Fign. 15 bis 18 zeigen eine für das Verständnis der Erfindung nützliche Ausführungsform; Die Fign. 15 bis 18 entsprechen jeweils den Fign. 11 bis 14. Im Unterschied zur Ausführungsform der Fig. 11 bis 14 sind bei dem Ausführungsbeispiel die LEDs wieder auf einer Ebene senkrecht zur optischen Achse angebracht, wie in Fig. 4. Jedoch weist die Endoskopspitze zusätzlich zu der Endoskopspitze der Fig. 4 einen Ringspiegel auf, der um das Objektiv herum angebracht ist. Bevorzugt ist der Ringspiegel rotationssymmetrisch mit der optischen Achse des Objektivs als der Rotationsachse. Die spiegelnde Fläche des Ringspiegels ist den LEDs zugewandt und gegenüber der optischen Achse um einen endlichen Winkel geneigt. Zum Beispiel kann der Neigungswinkel in einem Bereich von 20° bis 70° bezogen auf die optische Achse sein. Dadurch wird ein Teil der Strahlung in den äußeren Bereich des Blickfelds reflektiert, so dass auch dort, und insbesondere auch bei Blickwinkeln, die größer als 180° sind, eine ausreichende Beleuchtung erzielt wird, wie in den Fign. 17 und 18 zu sehen ist.

Der Ringspiegel sollte bevorzugt so angebracht sein, dass er keine Abschattung zusätzlich zu der durch das Objektiv bewirkt. Das heißt, die Auskragung des Ringspiegels sollte abhängig von der Höhe in Richtung der optischen Achse, auf der der Ringspiegel angebracht ist, bestimmt werden.

Bevorzugt reflektiert der Ringspiegel Emissionslicht von den LEDs, das in der Endoskopspitze der Fig. 4 durch das Objektiv abgeschattet wird, und daher nicht oder nur unwesentlich zur Beleuchtung des Objektraums beiträgt. Dadurch wird die Lichtausnutzung verbessert, und der Energieverbrauch und die Wärmeentwicklung werden verringert.

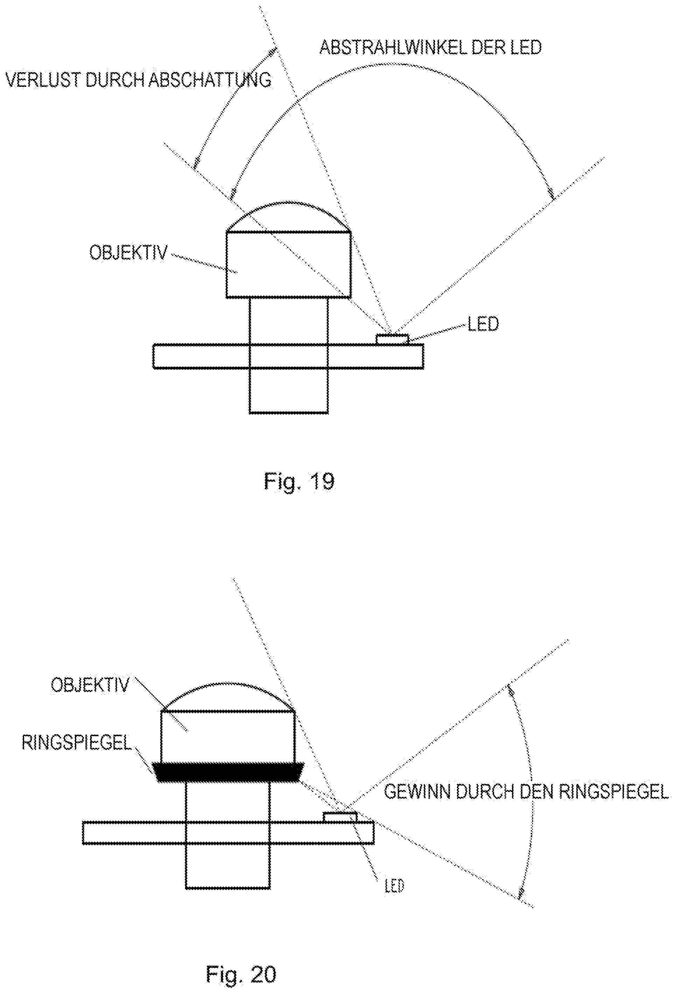

Dies ist in den Fign. 19 und 20 gezeigt. Fig. 19 zeigt eine Endoskopspitze (ohne die durchsichtige Kappe) gemäß dem Stand der Technik, bei dem ein Teil des Emissionslicht von der LED durch das Objektiv abgeschattet wird. Im Gegensatz dazu wird gemäß dem Ausführungsbeispiel des abgeschatteten Lichts durch den Ringspiegel in das äußere Blickfeld reflektiert, wie in Fig. 20 gezeigt. Die Abschattung in der Mitte ändert sich nicht.

Die Spiegelfläche des Ringspiegels bildet eine Kegelstumpffläche, bei der in Richtung der optischen Achse keine optische Brechkraft ausgeübt wird. Jedoch kann es in einigen Ausführungsbeispielen der Erfindung vorteilhaft sein, wenn der Ringspiegel in Richtung der optischen Achse eine optische Brechkraft ausübt, um Licht in bestimmte Bereiche des Blickfelds zu richten. In einem solchen Fall ist die Spiegelfläche in den Querschnitten der Fign. 15, 16, 17 und 20 gekrümmt. Die Spiegelfläche des Ringspiegels kann im allgemeinen sogar eine beliebige Form haben, welche nicht Teil des Schutzumfangs des Anspruchs 1 ist. Bevorzugt (aber nicht notwendigerweise) fällt die Achse des Kegelstumpfs mit der optischen Achse des Objektivs zusammen.

In einigen Ausführungsbeispielen der Erfindung ist zusätzlich noch die Unterseite des Ringspiegels und zumindest ein Teil der äußeren Hülle des Objektivs, der sich an die Unterseite des Ringspiegels anschließt, verspiegelt. Dadurch kann Emissionslicht, das mit einem flachen Winkel aus der LED austritt, durch Mehrfachreflektion an der äußeren Hülle des Objektivs und der Unterseite des Ringspiegels in das äußere Blickfeld gelenkt werden. Dies erhöht die Lichtausnutzung noch weiter.

Der gleiche Effekt kann auch ohne den Ringspiegel erzielt werden, wenn die äußere Hülle des Objektivs ein auskragendes Teil oberhalb der LEDs in Richtung der optischen Achse aufweist, wie z.B. in den Fign. 4 und 11. Gemäß einem dritten Ausführungsbeispiel der Erfindung sind sowohl die Unterseite der Auskragung als auch zumindest ein Teil der äußeren Hülle des Objektivs, der sich unterhalb der Auskragung an die Auskragung anschließt, verspiegelt, um Licht von der LED in das äußere Blickfeld zu lenken.

Gemäß der Erfindung ist sowohl die Abstrahlrichtung der LEDs gegenüber der optischen Achse des Objektiv geneigt (wie in Fig. 11) als auch ein Ringspiegel um das Objektiv angebracht (wie in Fig. 15). Zusätzlich kann noch ein Teil der äußeren Hülle des Objektivs verspiegelt sein, wie in dem dritten Ausführungsbeispiel. Dadurch lässt sich die Beleuchtung im äußeren Blickfeld noch weiter verbessern.

Soweit die äußere Hülle des Objektivs nicht verspiegelt ist, kann sie schwarz sein, so dass sie nahezu kein Licht reflektiert.

Die reflektierenden Flächen können durch metallische Beschichtung, z.B. mit Silber, gebildet werden. Alternativ kann in einem Fall, in dem der Raum zwischen der Kappe, der Außenhülle des Objektivs und der Montagefläche der LEDs kann mit einem durchsichtigen Dielektrikum gefüllt ist, eine reflektierende Fläche auch durch ein anderes Dielektrikum mit niedrigerer Brechzahl gebildet werden. Ferner kann in diesem Fall die Kappe identisch mit einer äußeren Schicht des durchsichtigen Dielektrikums sein.

Gemäß einer für das Verständnis der Erfindung nützlichen Ausführungsform wird ein optisch brechendes Element in den Raum zwischen den LEDs und der Kappe angebracht, um einen Teil des Lichts in das äußere Blickfeld zu lenken. Z.B. kann das optisch brechende direkt auf einem äußeren Teil der Lichtemissionsfläche der LED aufliegen und in einem Querschnitt keilförmig nach außen verlaufen. Beim Austritt des Lichts von der LED aus dem keilförmigen optisch brechenden Element wird es nach außen, hin zu dem äußeren Blickfeld, abgelenkt.

Die optisch brechenden Elemente der LEDs können verbunden sein, sodass sie eine Kegelstumpffläche bilden, wobei die Achse des Kegelstumpfs bevorzugt (aber nicht notwendigerweise) mit der optischen Achse des Objektivs zusammenfällt. Auch das optisch brechende Element des vierten Ausführungsbeispiels kann mit einem oder mehreren der ersten bis dritten Ausführungsbeispiele kombiniert werden.

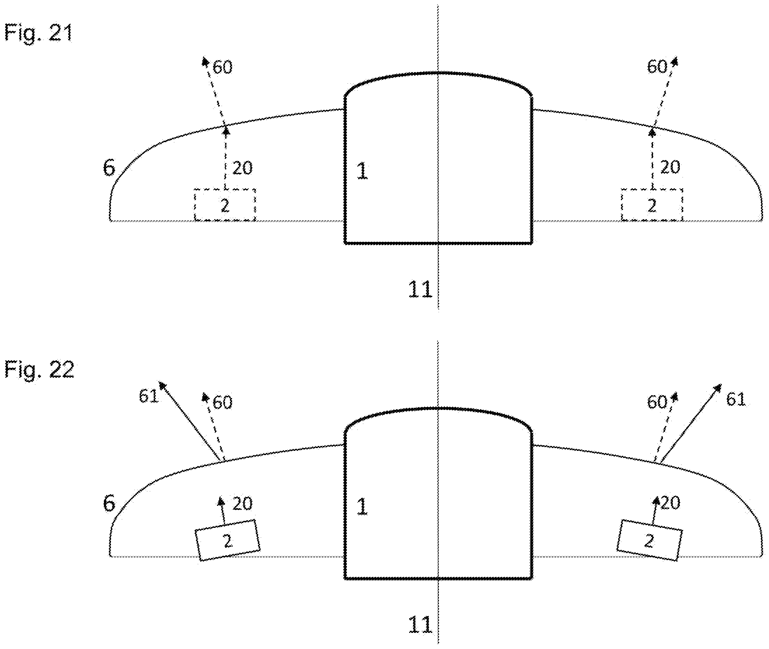

Die Fign. 21 und 22 illustrieren ein allgemeines Prinzip gemäß einigen Ausführungsbeispielen der Erfindung. Sie zeigen jeweils einen Querschnitt einer (hypothetischen oder realen) Endoskopspitze in einer Ebene, die von der optischen Achse 11 des Objektivs 1 und der Lichtemissionsachse von zumindest einer der LEDs 2 aufgespannt wird. Die LEDs 2 emittieren das (hypothetische oder reale) Emissionslicht 20 symmetrisch zu der Lichtemissionsachse.

Die Fig. 21 zeigt eine hypothetische Referenzkonfiguration, die der Fig. 4 gemäß dem Stand der Technik entspricht. In der Referenzkonfiguration sind die LEDs 2 in einer Ebene senkrecht zu der optischen Achse 11 des Objektivs 1 angeordnet. Die LEDs 2 emittieren (hypothetisches) Emissionslicht 20 parallel zu der Richtung der optischen Achse 11. Das (hypothetische) Emissionslicht 20 fällt direkt, d.h., ohne dass es von anderen Komponenten, wie z.B. Spiegeln oder optisch brechenden Elementen (Linsen) abgelenkt wird, auf die durchsichtige Kappe 6. Dort wird es möglicherweise abgelenkt und als hypothetisches Beleuchtungslicht 60 in den Objektraum (bzw. in das Blickfeld) des Objektivs 1 emittiert. Das hypothetische Beleuchtungslicht basiert ausschließlich auf Emissionslicht, das direkt von den LEDs 2 auf die Kappe 6 emittiert wird. Die gestrichelten Linien deuten an, dass die Referenzkonfiguration eine hypothetische Konfiguration ist.

Eine reale Konfiguration gemäß einigen Ausführungsbeispielen der Erfindung ist zum Vergleich in Fig. 22 gezeigt. Die reale Konfiguration unterscheidet sich von der Referenzkonfiguration dadurch, dass eine oder beide der folgenden Bedingungen erfüllt sind:
- Das Emissionslicht 20 wird von den LEDs 2 nicht parallel zu der optischen Achse 11 emittiert; und
- Das (reale) Beleuchtungslicht 61 basiert zumindest teilweise auf Emissionslicht, das nicht direkt von zumindest einer LED 2 auf die Kappe 6 emittiert wurde, sondern durch ein reflektierendes oder brechendes Element abgelenkt wurde.

Ansonsten ist die reale Konfiguration der Fig. 21 funktional identisch zu der hypothetischen Konfiguration der Fig. 22. Insbesondere ist der Austrittspunkt der jeweiligen Lichtemissionsachse aus den LEDs 2 an derselben Position.

Fig. 22 zeigt ein Beispiel in dem das Emissionslicht 20 nicht parallel zur optischen Achse emittiert wird. Zusätzlich wird dadurch, dass eine Lücke zwischen dem Emissionslicht 20 und dem Beleuchtungslicht 61 besteht, angedeutet, dass das Beleuchtungslicht andere Komponenten als die, die direkt von den LEDs 2 auf die Kappe 6 emittiert wurden, enthält.

Wie man aus Fig. 22 sehen kann, ist das reale Beleuchtungslicht 61 weiter von der optischen Achse weg gerichtet als das hypothetische Beleuchtungslicht 60.

Die Pfeile 20, die das Emissionslicht der LEDs 2 symbolisieren, geben den Schwerpunt der Winkelverteilung des Eimissionslichts an. Typischerweise wird das Emissionslicht symmetrisch um diesen Schwerpunkt emittiert (z.B. Lambertscher Strahler). Die Pfeile 60 und 61, die das (hypothetische bzw. reale) Beleuchtungslicht symbolisieren, geben den Schwerpunkt der Winkelverteilung des jeweiligen Beleuchtungslichts an. Im Allgemeinen ist die Winkelverteilung des Beleuchtungslichts nicht notwendigerweise symmetrisch um diesen Pfeil wegen der Abschattung durch das Objektiv und, in dem Fall des realen Beleuchtungslichts 61, unter Umständen auch wegen der gespiegelten oder optisch gebrochenen oder optisch gebeugten Komponenten des Beleuchtungslichts.

Eine LED ist ein Beispiel für eine Lichtemissionseinrichtung gemäß einigen Ausführungsbeispielen der Erfindung. Anstelle einer LED kann z.B. auch ein Emissionsende eines Lichtleiters verwendet werden. Es können auch einige der Lichtemissionseinrichtungen LEDs und andere der Lichtemissionseinrichtungen Emissionsenden von Lichtleitern sein.

Unter einem Objektiv wird eine Linse oder ein System von Linsen und evtl. weiteren optischen Elementen verstanden, die eine Szene auf eine Bildfläche abbilden. Insbesondere bildet ein Objektiv die Szene stetig auf die Bildfläche ab. Das heißt, in der Szene benachbarte Punkte sind auch in dem Bild auf der Bildfläche benachbart. Der Blickwinkel des Objektivs ist größer als 180°. Bevorzugt ist er größer als 200°, noch bevorzugter größer als 220°, und noch weiter bevorzugt größer als 230°. Solche Objektive sind zum Beispiel in EP19187218.3 beschrieben. Typischerweise weist die Endoskopspitze ein einziges Objektiv auf.

Das Endoskop kann ein starres Endoskop sein, bei dem das proximale Ende der Endoskopspitze mit einem starren Rohr verbunden ist. Das Endoskop kann ein flexibles Endoskop sein, bei dem das proximale Ende der Endoskopspitze mit einem flexiblen Schlauch verbunden ist. Sowohl das starre Rohr als auch der flexible Schlauch werden als "Schaft" bezeichnet.

Die Verbindung der Endoskopspitze mit dem Schaft kann direkt oder indirekt mittels einem Angulationselement erfolgen. Das Endoskop kann ein frei schwebendes Endoskop (Kapselendoskop) sein, das keinen Schaft besitzt. Das Endoskop (und damit natürlich auch die Endoskopspitze) kann zum Einführen in einen Hohlraum eines menschlichen Körpers geeignet sein, wie z.B. ein Bronchoskop, ein Laryngoskop, oder ein Coloskop.

## Patentansprüche

1. Endoskopspitze (10) oder Kapselendoskop mit
einem Objektiv (1) zum Abbilden eines Blickfelds; und
einer Beleuchtungsvorrichtung zum Beleuchten des Blickfelds mit Beleuchtungslicht (61), wobei
das Objektiv (1) eine optische Achse (11) hat;
das Objektiv (1) einen Blickwinkel, der größer als 180° ist, hat;
die Beleuchtungsvorrichtung in einer Draufsicht entlang der optischen Achse (11) um das Objektiv (1) herum angeordnet ist;
die Beleuchtungsvorrichtung eine durchsichtige Kappe (6) aufweist, aus der das Beleuchtungslicht (61) in das Blickfeld emittiert wird;
die Beleuchtungsvorrichtung eine oder mehrere Lichtemissionseinrichtungen (2) aufweist, die konfiguriert sind, jeweiliges Emissionslicht (20) aus einer jeweiligen Lichtemissionsfläche zu emittieren; und
zumindest eine der Lichtemissionseinrichtungen (2) so konfiguriert ist, dass ein Schwerpunkt einer Winkelverteilung des jeweiligen Emissionslichts (20) in einer Richtung, die um einen Winkel, der größer oder gleich 5° und kleiner oder gleich 85° ist, weg von der optischen Achse (11) gekippt ist, liegt,
wobei die Beleuchtungsvorrichtung für jede der Lichtemissionseinrichtungen (2) eine jeweilige reflektierende Fläche aufweist, die zumindest einen Teil des jeweiligen Emissionslichts (20) in eine jeweilige Richtung reflektiert, die weiter weg von der Richtung der optischen Achse (11) ist als eine jeweilige Richtung, in der der Teil des jeweiligen Emissionslichts (20) auf die jeweilige reflektierende Fläche trifft,
wobei die Beleuchtungsvorrichtung rotationssymmetrisch um eine Rotationsachse ist, die jeweiligen reflektierenden Flächen Teil eines Ringspiegels sind und der Ringspiegel einen Teil einer ersten Kegelstumpffläche bildet, wobei die Achse der ersten Kegelstumpffläche vorzugsweise identisch mit der optischen Achse (11) ist.

2. Die Endoskopspitze (10) bzw. das Kapselendoskop nach Anspruch 1, wobei jede der Lichtemissionseinrichtungen (2) so konfiguriert ist, dass der Schwerpunkt der Winkelverteilung des jeweiligen Emissionslichts (20) in eine jeweilige Richtung weg von der optischen Achse (11) liegt.

3. Die Endoskopspitze (10) bzw. das Kapselendoskop nach Anspruch 1 oder 2, wobei
die Beleuchtungsvorrichtung so angeordnet ist, dass die Rotationsachse identisch mit der optischen Achse (11) ist.

4. Die Endoskopspitze (10) bzw. das Kapselendoskop nach einem der Ansprüche 1 bis 3, wobei,
die Lichtemissionseinrichtungen (2) auf einer zweiten Kegelstumpffläche angeordnet sind.

5. Die Endoskopspitze (10) bzw. das Kapselendoskop nach Anspruch 4, wobei
die Achse der zweiten Kegelstumpffläche identisch mit der optischen Achse (11) ist.

6. Die Endoskopspitze (10) bzw. das Kapselendoskop nach einem der Ansprüche 1 bis 5, wobei eine Winkelverteilung des Beleuchtungslichts (61) in einer Ebene, die die optische Achse (11) enthält, einen Winkelbereich von mehr als 90° umfasst.

7. Die Endoskopspitze (10) bzw. das Kapselendoskop nach einem der Ansprüche 1 bis 6, wobei jede der Lichtemissionseinrichtungen (2) eine lichtemittierende Diode oder ein Emissionsende eines Lichtleiters ist.

8. Die Endoskopspitze (10) bzw. das Kapselendoskop nach einem der Ansprüche 1 bis 7, wobei
sich das Objektiv (1) am distalen Ende der Endoskopspitze (10) befindet;
die Lichtemissionseinrichtungen (2) in einer Anordnungsebene, die senkrecht zu der optischen Achse (11) liegt, angeordnet sind; und
die Anordnungsebene weiter von dem distalen Ende der Endoskopspitze (10) entfernt ist als ein Apex einer Linse des Objektivs (1), die dem Blickfeld des Objektivs (1) am nächsten ist.

9. Die Endoskopspitze (10) bzw. das Kapselendoskop nach Anspruch 8, wobei
eine äußere Hülle des Objektivs (1) eine Auskragung besitzt, die in Richtung der optischen Achse (11) näher an dem distalen Ende als die Lichtemissionseinrichtungen (2) angeordnet ist;
eine Unterseite der Auskragung, die den Lichtemissionseinrichtungen (2) zugewandt ist, und zumindest ein Teil der äußeren Hülle, der sich in proximaler Richtung an die Unterseite der Auskragung anschließt, verspiegelt sind.

10. Die Endoskopspitze (10) bzw. das Kapselendoskop nach einem der Ansprüche 1 bis 9, wobei das Objektiv (1) das einzige Objektiv ist, das in der Endoskopspitze (10) vorhanden ist.

11. Endoskop, das aufweist:
die Endoskopspitze (10) nach einem der Ansprüche 1 bis 10;
einen Schaft, dessen distales Ende direkt oder indirekt mit einem proximalen Ende der Endoskopspitze (10) verbunden ist.

## Claims

1. An endoscope tip (10) or capsule endoscope comprising
an objective (1) for imaging a field of view; and
an illumination device for illuminating the field of view with illumination light (61), wherein
the objective (1) has an optical axis (11);
the objective (1) has an angle of view greater than 180°;
the illumination device is arranged around the objective (1) in a plan view along the optical axis (11);
the illumination device comprises a transparent cap (6) from which the illumination light (61) is emitted into the field of view;
the illumination device comprises one or more light emitting devices (2) configured to emit respective emission light (20) from a respective light emitting surface; and
at least one of the light emitting devices (2) is configured such that a centroid of an angular distribution of the respective emission light (20) lies in a direction tilted away from the optical axis (11) by an angle greater than or equal to 5° and less than or equal to 85°,
wherein the illumination device comprises, for each of the light emitting devices (2), a respective reflecting surface that reflects at least a part of the respective emission light (20) in a respective direction further away from the direction of the optical axis (11) than a respective direction in which the part of the respective emission light (20) impinges on the respective reflecting surface,
wherein the illumination device is rotationally symmetric about an axis of rotation, the respective reflecting surfaces are part of a ring mirror, and the ring mirror forms part of a first truncated cone surface, wherein the axis of the first truncated cone surface is preferably identical to the optical axis (11).

2. The endoscope tip (10) or capsule endoscope according to claim 1, wherein each of the light emitting devices (2) is configured such that the centroid of the angular distribution of the respective emission light (20) lies in a respective direction away from the optical axis (11).

3. The endoscope tip (10) or capsule endoscope according to claim 1 or 2, wherein
the illumination device is arranged such that the axis of rotation is identical to the optical axis (11).

4. The endoscope tip (10) or capsule endoscope according to any one of claims 1 to 3, wherein
the light emitting devices (2) are arranged on a second truncated cone surface.

5. The endoscope tip (10) or capsule endoscope according to claim 4, wherein
the axis of the second truncated cone surface is identical to the optical axis (11).

6. The endoscope tip (10) or capsule endoscope according to any one of claims 1 to 5, wherein an angular distribution of the illumination light (61) in a plane containing the optical axis (11) comprises an angular range of more than 90°.

7. The endoscope tip (10) or capsule endoscope according to any one of claims 1 to 6, wherein each of the light emitting devices (2) is a light emitting diode or an emission end of a light guide.

8. The endoscope tip (10) or capsule endoscope according to any one of claims 1 to 7, wherein
the objective (1) is located at the distal end of the endoscope tip (10);
the light emitting devices (2) are arranged in an arrangement plane perpendicular to the optical axis (11); and
the arrangement plane is farther from the distal end of the endoscope tip (10) than an apex of a lens of the objective (1) closest to the field of view of the objective (1).

9. The endoscope tip (10) or capsule endoscope according to claim 8, wherein
an outer casing of the objective (1) has a projection arranged closer to the distal end than the light emitting devices (2) in the direction of the optical axis (11);
a bottom of the projection facing the light emitting devices (2) and at least a part of the outer casing adjoining the bottom of the projection in the proximal direction are reflective.

10. The endoscope tip (10) or capsule endoscope according to any one of claims 1 to 9, wherein the objective (1) is the only objective present in the endoscope tip (10).

11. An endoscope comprising:
the endoscope tip (10) according to any one of claims 1 to 10;
a shaft whose distal end is directly or indirectly connected to a proximal end of the endoscope tip (10).

## Revendications

1. Pointe d'endoscope (10) ou endoscope de type capsule comprenant
un objectif (1) pour imager un champ de vision ; et
un dispositif d'éclairage pour éclairer le champ de vision avec une lumière d'éclairage (61), dans lequel
l'objectif (1) a un axe optique (11) ;
l'objectif (1) a un angle de vision supérieur à 180° ;
le dispositif d'éclairage est agencé autour de l'objectif (1) dans une vue en plan le long de l'axe optique (11) ;
le dispositif d'éclairage comprend un capuchon transparent (6) à partir duquel la lumière d'éclairage (61) est émise dans le champ de vision ;
le dispositif d'éclairage comprend un ou plusieurs dispositifs d'émission de lumière (2) configurés pour émettre une lumière d'émission respective (20) à partir d'une surface d'émission de lumière respective ; et
au moins l'un des dispositifs d'émission de lumière (2) est configuré de sorte qu'un centre de gravité d'une distribution angulaire de la lumière d'émission respective (20) se trouve dans une direction inclinée à l'écart de l'axe optique (11) d'un angle supérieur ou égal à 5° et inférieur ou égal à 85°,
dans lequel le dispositif d'éclairage comprend, pour chacun des dispositifs d'émission de lumière (2), une surface réfléchissante respective qui réfléchit au moins une partie de la lumière d'émission respective (20) dans une direction respective qui est plus éloignée de la direction de l'axe optique (11) qu'une direction respective dans laquelle la partie de la lumière d'émission respective (20) frappe la surface réfléchissante respective,
dans lequel le dispositif d'éclairage est symétrique en rotation autour d'un axe de rotation, les surfaces réfléchissantes respectives font partie d'un miroir annulaire, et le miroir annulaire fait partie d'une première surface tronconique, dans lequel l'axe de la première surface tronconique est de préférence identique à l'axe optique (11).

2. Pointe d'endoscope (10) ou endoscope de type capsule selon la revendication 1, dans lequel chacun des dispositifs d'émission de lumière (2) est configuré de sorte que le centre de gravité de la distribution angulaire de la lumière d'émission respective (20) se trouve dans une direction respective éloignée de l'axe optique (11).

3. Pointe d'endoscope (10) ou endoscope de type capsule selon la revendication 1 ou 2, dans lequel
le dispositif d'éclairage est agencé de sorte que l'axe de rotation est identique à l'axe optique (11).

4. Pointe d'endoscope (10) ou endoscope de type capsule selon l'une quelconque des revendications 1 à 3, dans lequel
les dispositifs d'émission de lumière (2) sont agencés sur une seconde surface tronconique.

5. Pointe d'endoscope (10) ou endoscope de type capsule selon la revendication 4, dans lequel
l'axe de la seconde surface tronconique est identique à l'axe optique (11).

6. Pointe d'endoscope (10) ou endoscope de type capsule selon l'une quelconque des revendications 1 à 5, dans lequel une distribution angulaire de la lumière d'éclairage (61) dans un plan contenant l'axe optique (11) comprend une plage angulaire supérieure à 90°.

7. Pointe d'endoscope (10) ou endoscope de type capsule selon l'une quelconque des revendications 1 à 6, dans lequel chacun des dispositifs d'émission de lumière (2) est une diode électroluminescente ou une extrémité d'émission d'un guide de lumière.

8. Pointe d'endoscope (10) ou endoscope de type capsule selon l'une quelconque des revendications 1 à 7, dans lequel
l'objectif (1) est situé à l'extrémité distale de la pointe d'endoscope (10) ;
les dispositifs d'émission de lumière (2) sont agencés dans un plan d'agencement qui est perpendiculaire à l'axe optique (11) ; et
le plan d'agencement est plus éloigné de l'extrémité distale de la pointe d'endoscope (10) qu'un sommet d'une lentille de l'objectif (1) qui est le plus proche du champ de vision de l'objectif (1).

9. Pointe d'endoscope (10) ou endoscope de type capsule selon la revendication 8, dans lequel
une gaine externe de l'objectif (1) a une saillie qui est agencée plus près de l'extrémité distale que les dispositifs d'émission de lumière (2) dans la direction de l'axe optique (11) ;
une surface inférieure de la saillie qui fait face aux dispositifs d'émission de lumière (2) et au moins une partie de la gaine externe qui est contiguë à la surface inférieure de la saillie dans la direction proximale sont réfléchissantes.

10. Pointe d'endoscope (10) ou endoscope de type capsule selon l'une quelconque des revendications 1 à 9, dans lequel l'objectif (1) est le seul objectif présent dans la pointe d'endoscope (10).

11. Endoscope comprenant :
la pointe d'endoscope (10) selon l'une quelconque des revendications 1 à 10 ;
un arbre dont l'extrémité distale est reliée directement ou indirectement à une extrémité proximale de la pointe d'endoscope (10).
